# EUROPEAN PATENT APPLICATION

(11) **EP 3 878 482 A1**
(43) Date of publication of application: **15.09.2021**
(21) Application number: 20162311.3
(22) Date of filing: 11.03.2020
(51) Int. Cl.: A61L 15/58, A61L 24/04, C09J 5/00, C09J 7/00, C09J 9/02, C09J 11/06

(54) **ELECTRICALLY SWITCHABLE ADHESIVES FOR APPLICATION ON SKIN AND RELATED PRODUCTS AND USES**

(71) Applicant: Nitto Belgium NV, 3600 Genk (BE)
(72) Inventor: HOEBERS, Charly, 3600 Genk (BE); PLESSERS, Senne, 3600 Genk (BE)
(74) Representative: Grünecker Patent- und Rechtsanwälte PartG mbB

(57) **Abstract**

Medical devices and pressure-sensitive adhesive sheet or tape for application on skin comprising an electrically peelable pressure-sensitive adhesive for application on skin are disclosed, which comprise: an electrically conductive member, and a pressure-sensitive adhesive layer in direct contact with the electrically conductive member. Furthermore, an electrically peelable pressure-sensitive adhesive for application on skin is disclosed, the adhesive comprising: an electrically conductive member, and a pressure-sensitive adhesive layer in direct contact with the electrically conductive member, wherein the pressure-sensitive adhesive layer comprises a compound which dissociates into ionic species upon applying a voltage in the range of from 1 to 100 V to the electrically conductive member and wherein the pressure-sensitive adhesive layer exhibits an ionic conductivity of at least 10⁻¹¹ S/cm at 25°C with respect to the dissociated ionic species. In addition, related uses and methods are described. The invention enables maximized bonding strength to human skin and high resistance to perspiration, while simultaneously eliminating irritation and damage to the upper skin layer upon peeling.

## Description

### FIELD OF INVENTION

The present invention relates to medical devices and pressure-sensitive adhesive sheets or tapes comprising an electrically peelable pressure-sensitive adhesive for application on skin and the use of said adhesive in medical pressure-sensitive adhesive sheets or tapes, transdermal patches, wound dressings, band aids, or for affixing medical devices, wearables and cosmetic products onto skin.

### BACKGROUND OF THE INVENTION

A large number of medical adhesive compositions have been developed with the aim to maximize the bonding strength of the medical adhesive to human skin and simultaneously minimize irritation and damage to the upper skin layer. Exemplary medical adhesives are disclosed in EP 1 367 109 A1, US 2008/311396 A1 and US 2007/0275239 A1, for example. However, in conventional systems, further increasing the bonding strength in an attempt to increase wear times and/or fixation forces inevitably results in increased irritation, skin damage and/or so-called mechanical trauma upon removal.

In order to avoid such medical adhesive related skin injuries, several switchable medical adhesives have been developed, wherein a reduction in peel strength can be achieved in a controllable way and in a relatively short period of time by external triggers.

Examples of known switchable adhesives include those disclosed in US 5 387 450 A and US 5 156 911 A, where temperature changes are used to convert the adhesive from a tacky to a non-tacky state. However, such adhesives suffer from the risk of accidental debonding in case low temperature differences are applied or the risk of burns when applying high temperature differences.

EP 2 957 610 A1, US 2016/312087 A1 and US 2017/0081568 A1 disclose examples of adhesives which enable debonding on demand by applying UV light. However, when high dosages are needed to trigger the debonding, there is a risk of skin and/or DNA damage, whereas low dosages may lead to unwanted debonding. In addition, debonding may only be achieved with external UV light sources which may not be available at all times.

In US 4 331 576 A and US 5 032 637 A, medical adhesive compositions are proposed which enable debonding by the presence of water. However, these types of adhesives are not suited for long-term application on skin as release may be triggered by contact with sweat, for example.

In view of the above, it remains desirable to provide a medical adhesive composition which enables maximized bonding strength to human skin and high resistance to perspiration, while simultaneously reducing irritation and damage to the upper skin layer, which could in turn result in higher fixation forces, longer wear times of e.g. medical tapes, dressings, wearables and adhered devices, and ultimately improved quality of life for patients and users.

### SUMMARY OF THE INVENTION

The present invention solves these problems with the subject matter of the claims as defined herein. Further advantages of the present invention will be further explained in detail in the section below.

According to a first aspect of the present invention, a medical device is provided, which comprises an electrically peelable pressure-sensitive adhesive for application on skin, the adhesive comprising: an electrically conductive member, and a pressure-sensitive adhesive layer in direct contact with the electrically conductive member. Where the closest prior art uses debonding triggers such as water, UV light or temperature differences, the invention described here uses electricity to greatly reduce the peel strength of the medical adhesive. More specifically, a very small electrical current may be used to apply a potential difference between the substrate and the adhesive sheet. Accordingly, the non-tacky state of the adhesive can be achieved in a very controllable way and in a relatively short period of time. In preferred embodiments, the medical device may comprise a pressure-sensitive adhesive sheet or tape according to the second aspect or an electrically peelable pressure-sensitive adhesive according to the third aspect described in the following.In a second aspect of the present invention, a pressure-sensitive adhesive sheet or tape for application on skin is provided, the sheet or tape comprising: an electrically peelable pressure-sensitive adhesive comprising: an electrically conductive member, and a pressure-sensitive adhesive layer in direct contact with the electrically conductive member,, and one or more additional conductive members that are not in electrical contact with any of the pressure-sensitive adhesive layer and the electrically conductive member, wherein the one or more additional conductive members are provided so as to establish electrical contact to the skin upon applying the sheet or tape. Thus, an electrically switchable medical adhesive sheet that can be easily peeled off without any irritation or skin damage after is provided. In preferred embodiments, the pressure-sensitive adhesive sheet or tape according to the second aspect may comprise an electrically peelable pressure-sensitive adhesive according to the third aspect described in the following.

In a third aspect, the present invention relates to an electrically peelable pressure-sensitive adhesive for application on skin, comprising: an electrically conductive member, and a pressure-sensitive adhesive layer in direct contact with the electrically conductive member, wherein the pressure-sensitive adhesive layer comprises a compound which dissociates into ionic species upon applying a voltage in the range of from 1 to 100 V to the electrically conductive member and wherein the pressure-sensitive adhesive layer exhibits an ionic conductivity of at least 10⁻¹¹ S/cm at 25°C with respect to the dissociated ionic species.

A fourth aspect of the present invention relates to the use of the electrically peelable pressure-sensitive adhesive according to the third aspect in medical pressure-sensitive adhesive sheets or tapes, transdermal patches, wound dressings, band aids, or for affixing medical devices, wearables and cosmetic products onto skin.

In a fifth aspect, the present invention relates to a method of applying a medical device according to the first aspect or a pressure-sensitive adhesive sheet or tape on skin according to the second aspect, comprising the subsequent steps of: (a) adhering the medical device according to the first aspect or the pressure-sensitive adhesive sheet or tape according to the second aspect on skin via the pressure-sensitive adhesive layer and establishing contact between the one or more additional conductive members and the skin surface, wherein a voltage source is either integrated into the medical device according to the first aspect or the pressure-sensitive adhesive sheet or tape according to the second aspect or independently provided and connected so that a first electrode of the voltage source is connected with the electrically conductive member and a second electrode of the voltage source is connected with the one or more additional conductive members, the first and second electrodes having opposite polarities; (b) activating the voltage source so as to establish a potential difference of 1 V to 100 V along the thickness direction of the pressure-sensitive adhesive layer; (c) peeling off the medical device according to the first aspect or the pressure-sensitive adhesive sheet or tape according to the second aspect from the skin surface; and (d) optionally re-adhering the medical device according to the first aspect or the pressure-sensitive adhesive sheet or tape to the skin according to the second aspect and subsequently deactivating or adjusting the voltage source to establish a potential difference of less than 1 V along the thickness direction of the pressure-sensitive adhesive layer.

Preferred embodiments of the medical devices, sheets and tapes, and the electrically peelable pressure-sensitive adhesives according to the present invention and other aspects of the present invention are described in the following description and the claims.

### BRIEF DESCRIPTION OF THE DRAWINGS

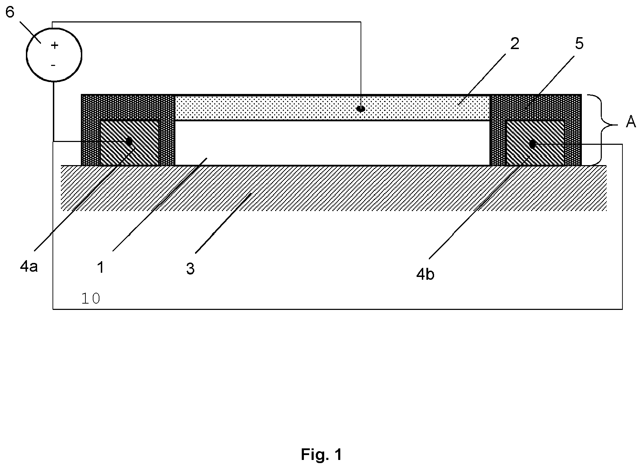

FIG. 1 is a schematic representation of an exemplary configuration of a pressure-sensitive adhesive sheet or tape for application on skin according to the second embodiment of the present invention.

### DETAILED DESCRIPTION OF THE INVENTION

For a more complete understanding of the present invention, reference is now made to the following description of the illustrative embodiments thereof:

### Electrically Peelable Pressure-Sensitive Adhesive

In a first embodiment, the present invention relates to an electrically peelable pressure-sensitive adhesive for application on skin, comprising: an electrically conductive member, and a pressure-sensitive adhesive layer in direct contact with the electrically conductive member; wherein the pressure-sensitive adhesive layer comprises a compound which dissociates into ionic species upon applying a voltage to the electrically conductive member, i.e. a DC voltage in the range of from 1 to 100 V, preferably from 1 to 20 V, more preferably from 1 to 10 V, especially preferably 1 to 5 V; and wherein the pressure-sensitive adhesive layer exhibits an ionic conductivity of at least 10⁻¹¹ S/cm at 25°C with respect to the dissociated ionic species.

The outermost layer of the epidermis (i.e. the stratum corneum) and hair both exhibit a negatively charged surface at physiological pH. Upon adhering the electrically peelable pressure-sensitive adhesive on skin, applying a potential difference between the conductive member and the skin surface along the thickness direction will affect dissociation of the compound and migration of cationic species towards the skin surface, which typically results in a liquid-like interface between the adhesive layer and the skin surface and substantially reduces the peel strength.

The applied potential difference is not particularly limited, but is preferably less than 100 V, more preferably 10 V or less and further preferably 5V or less, enabling use of a large variety of low voltage sources, such as printed batteries and wireless power transfer using NFC or Qi standards, as will be further illustrated below.

Electrically switchable adhesion properties of the pressure-sensitive adhesive layer are generally achieved by mixing of a compound capable of dissociating in to an ionic species with low viscosity properties into the adhesive. Conventionally, the performance of medical adhesives tends to be a compromise between appropriate initial tack, firm bonding strength and sustained adhesion on one hand, and peeling ability (especially after prolonged wearing times) and repositionability without causing trauma to the wound and surrounding skin on the other. Advantageously, in the present invention, the adhesive composition is not particularly limited and may be selected from compositions enabling high adhesive strength to skin without having to take into account the peeling ability and potential skin damage or irritation, and also providing the opportunity to increase the wearing time of medical tapes to unprecedented levels and to improve patient comfort and confidence during usage.

In particular, as long as it is safe for skin application (i.e. non-irritant, non-sensitizing and biocompatible, e.g. according to the ISO 10993 standard) and the ion conductivity of the PSA layer is maintained, the adhesive as such is not particularly limited and may include a polymerized product or a partially polymerized product of monomers, for example. The monomers may be one kind of monomers and also be a monomer mixture of two or more kinds of monomers. Exemplary adhesive polymers include, but are not limited to one or more selected from acrylic polymers, rubber-based polymers, vinyl alkyl ether-based polymers, silicone-based polymers, polyester-based polymers, polyamide-based polymers, urethane-based polymers, and fluorine-based polymers, for example. In particularly preferred embodiments, the pressure-sensitive adhesive layer comprises an adhesive (co-)polymer selected from one or more of an acrylic (co-)polymer, a silicone-based (co-)polymer or a urethane-based polymer.

In preferred embodiments, a content of the compound which dissociates into ionic species is within the range of 0.001 to 30 % by weight based on the total weight of the pressure-sensitive adhesive layer, preferably from 0.003 to 25 % by weight.

Since a smaller molecular weight will result in an increased ion mobility towards the negatively charged side (i.e. the PSA layer/skin interface) and hence facilitate the peel ability when applying a potential difference, the ionic species preferably comprise a cation having a molecular weight of 250 g/mol or less, further preferably 200 g/mol or less, and particularly preferably 160 g/mol or less.

A mixing amount of the compound with respect to the adhesive composition is not particularly limited. However, in order to effectively exert the function of the present invention, it is preferably 0.1 parts by weight or more and 40 parts by weight or less with respect to 100 parts by weight of the adhesive (co-)polymer, further preferably 0.3 parts by weight or more and 35 parts by weight or less, and especially preferably 0.5 parts by weight or more and 30 parts by weight or less.

In preferred embodiments, the compound which dissociates into ionic species upon applying a voltage to the electrically conductive member is an ionic liquid, further preferably an ionic liquid which is liquid or at least exhibits a very low viscosity behaviour at room temperature (approx. 23°C).

Suitable ionic liquids are not particularly limited but preferably comprise a cation selected from a nitrogen-containing onium cation, a sulfur-containing onium cation, or a phosphorus-containing onium cation. Particularly preferred cations include imidazolium-based cations, ammonium-based cations or pyridinium-based cations. Anions may be suitably selected from species compatible with the respective cation to provide liquid-like behavior at room temperature. Examples thereof include, but are not limited to amide-based anions, sulfonate-based anions, triflate-based anions, acetate-based anions and lactate-based anions. In addition, ionic liquids may also be modified for biocompatibility. Examples thereof include, but are not limited to, ionic liquids based on derivatives of amino acids, carbohydrates, cholinium, lactates, acesulfamate, and levulinate, for example.

In preferred embodiments, the adhesive layer exhibits an ionic conductivity of at least 10⁻⁷ S/cm at 25°C with respect to the dissociated ionic species, preferably at least 10⁻⁶ S/cm or more and 10⁻¹ S/cm or less. An upper limit of the ionic conductivity is preferably 10⁻² S/cm and further preferably 5·10⁻¹ S/cm, and a lower limit thereof is more preferably 10⁻⁵ S/cm and further preferably 10⁻⁴ S/cm. Within the given ranges, excellent peelability can be obtained even at low voltages. The ionic conductivity can be generally measured by an AC impedance method according to procedures known in the art. In embodiments, the adhesive layer may exhibit a resistance along the thickness direction of 0.1 MΩ or more, such as 1 MΩ or more, 10 MΩ or more, 100 MΩ or more, or 400 MΩ or more.

The ionic conductivity of the adhesive layer may be controlled by techniques known in the art. For example, the ion conductivity may be enhanced by suitably modifying the above-mentioned adhesive (co-)polymers to incorporate regions of high ionic conductivity (e.g. by graft or block co-polymerization). Moreover, electrolytes that are ionically conductive and capable of supporting ion diffusion of a salt solvated therein may be used in combination with any of the above-mentioned adhesive (co-)polymers. Suitable electrolytes include, but are not limited to ionically solvating molecules, including a plasticizer, or an oligomer or polymer also capable of solvating ions. As further examples, complex ion salts may be added. Suitable salts include alkali metal, alkaline earth and ammonium salts, for example. Further preferred salts include polyatomic, high dissociation constant anions, such as hexafluorophosphate, tetrafluoroborate, hexafluoroantimonate and perchlorate. As a further example of modifying the ion conductivity, the addition of polyethylene glycol to the adhesive layer formulation in order to assist the movement of the ionic liquid when applying a voltage to the conductive member may be mentioned. In preferred embodiments, polyethylene glycol having a number average molecular weight of 200 to 6000 is employed. Preferably, polyethylene glycol is added in a content of 0.001 to 30 % by weight based on the total weight of the pressure-sensitive adhesive layer.

In addition, water may be added to the adhesive while applying a voltage to promote the movement of the cations toward the adherend (skin) side.

In the present invention, a conductive filler may be added to the adhesive layer to impart conductivity. The conductive filler is not particularly limited, and a commonly known or commonly used conductive filler can be used. For example, graphite, carbon black, carbon fiber, metal powder such as silver and copper can be used. A content of the conductive filler is preferably 0.1% weight or more and 70% by weight or less based on the total weight of the pressure-sensitive adhesive layer. The conductive filler may be present in a varying concentration (i.e. gradient) in the thickness direction of the PSA layer.

In the present invention, as needed, within a range of not impairing effects of the present invention, various additives known in the art may be used, such as bioactive agents (e.g. antimicrobial agents, antiviral agents, anticancer agents, pain relievers, analgesics, anti-inflammatory agents, blood-clotting agents, nicotine or anesthetic agents, for example), natural preservatives, skin protectants, fillers, plasticizers, antioxidants, anti-aging agents, pigments, flame retardants, solvents, surfactants, and tackifying resins for example. The additives can be used alone and two or more kinds thereof can be used in combination.

A total content (mixing amount) of the additive is not particularly limited, but is preferably 0% weight or more and 20 % by weight or less based on the total weight of the pressure-sensitive adhesive layer.

The thickness of the pressure-sensitive adhesive layer is not particularly limited and may be suitably selected by the skilled artisan depending on the purpose of the product and the constituents and performance of the adhesive composition. Typically, the adhesive layer thickness ranges from 10 µm to 10 mm.

The electrically conductive member in direct contact with the pressure-sensitive adhesive layer (also referred to as first conductive member hereinbelow) typically serves as a connecting terminal for the voltage source serving to establish a potential difference along the thickness direction of the pressure-sensitive adhesive layer. In preferred embodiments, the first electrically conductive member is configured in the form of a sheet, foil or plate in direct contact with the surface of the pressure-sensitive adhesive layer opposed to the adherend, which enables even distribution of the applied voltage throughout the area of the adhesive layer. Alternatively or in addition, the first electrically conductive member may be configured in the form of a pattern over the adhesive layer (which may be printed circuits, such as printed circuit boards (PCBs) or flexible printed circuits (FPCs), for example). The thickness of the electrically conductive member when formed as a sheet, foil, plate or pattern or trace is not particularly limited and may be suitably selected depending on the materials, the manufacturing procedure and the desired flexibility. Typical thicknesses will range from 10 nm to 2 mm. Provided that a connection with the voltage source is enabled, the first electrically conductive member may also be configured in the form of conductive particles that may be partially or fully embedded in the pressure-sensitive adhesive layer. In preferred embodiments, the electrically conductive member comprises a metal (e.g., aluminum, copper, silver, iron), metal oxide (e.g. indium-tin-oxide (ITO)), carbon, a conductive polymer, and a combination thereof.

### Pressure-Sensitive Adhesive Sheet or Tape

In a second embodiment, the present invention relates to a pressure-sensitive adhesive sheet or tape for application on skin, comprising: an electrically peelable pressure-sensitive adhesive comprising: an electrically conductive member, a pressure-sensitive adhesive layer in direct contact with the electrically conductive member, and one or more additional conductive members that are not in electrical contact with any of the pressure-sensitive adhesive layer and the electrically conductive member, wherein the one or more additional conductive members are provided so as to establish electrical contact to the skin (directly or indirectly (e.g., via a conductive gel or liquid)) upon applying the sheet or tape.

Prefeably, the electrically peelable pressure-sensitive adhesive according to the first embodiment is used as the electrically peelable pressure-sensitive adhesive in the second embodiment.

The one or more additional conductive members may serve as connection terminals for a voltage source at an electrode (i.e. cathode) thereof that has the opposite polarity of the electrode (i.e. anode) connected to the conductive member in direct contact with the pressure-sensitive adhesive layer. Accordingly, the potential difference along the thickness direction of the pressure-sensitive adhesive layer may be established in a reliable and effective manner and the migration of cationic species towards the skin surface may be further promoted. As long as electrical contact to the skin may be established, the geometry of the one or more additional conductive members is not particularly limited and may include dotted patterns, grid patterns, track patterns or combinations thereof, for example.

While not being limited thereto, an example of such a pressure-sensitive adhesive sheet or tape is illustrated in Fig. 1. Specifically, Fig. 1 shows an exemplary electrically peelable pressure-sensitive adhesive sheet or tape (A) comprising a pressure-sensitive adhesive layer (1), onto which a first conductive member (2) configured as a conductive sheet, foil or plate is provided. As additional conductive members, the second and third conductive members (4a, 4b) are provided flush to the lower surface of the pressure-sensitive adhesive layer (1), which enables electrical contact with the surface of the skin (3) upon applying the tape or sheet thereon. One or more electrically insulating spacers (5) may be provided between the additional conductive members (4a, 4b) and any of the pressure-sensitive adhesive layer (1) and the electrically conductive member (2). In such a configuration, peel strength may be reversibly lowered by activating a voltage source, wherein a first electrode thereof is connected to the first conductive member (2) and a second electrode thereof is connected to the one or more additional conductive members (4a, 4b), the first and second electrodes having opposite polarities.

The pressure-sensitive adhesive sheet or tape according to the present invention may be provided as a carrierless sheet or tape (also known as transfer sheet or tape), a single-sided adhesive tape or sheet, or a double-sided adhesive tape or sheet including a carrier layer between two adhesive layers. In the latter case, the second adhesive layer may be composed of a different adhesive formulation and is not necessarily electrically switchable. Optionally, a release liner may be provided on the adhesive layer (or on both adhesive layers in the case of the double-sided adhesive tape. Generally, the optional release liner may be composed of materials known in the art, which may be suitably selected by the skilled artisan.

While the carrier material is not particularly limited, it may be preferred that the carrier layer comprises any of polyethylene, polypropylene, polyurethane, ethylene/propylene copolymers, ethylene/ethylacrylate copolymers, ethylene/vinyl acetate copolymers, silicone elastomers, polydimethylsiloxanes, neoprene rubber, polyisobutylene, polyacrylates, chlorinated poly-ethylene, polyvinyl chloride, vinyl chloride-vinyl acetate copolymer, crosslinked polymethacrylate polymers (hydrogel), polyvinylidene chloride, poly (ethylene terephthalate), butyl rubber, epichlorohydrin rubbers, ethylenevinyl alcohol copolymers, ethylene-vinyloxyethanol copolymers; silicone copolymers, for example, polysiloxane-polycarbonate copolymers, polysiloxanepolyethylene oxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxane-ethylenesilane copolymers), and the like; cellulose polymers, for example methyl or ethyl cellulose, hydroxy propyl methyl cellulose, and cellulose esters; polycarbonates; polytetrafluoro-ethylene; and the like. Particularly preferred are medical grade polyethers or polyester polyurethanes, thermoplastic polyester elastomer, perforated polyethylene, polypropylene and PET films, as well as medical grade woven or non-woven materials. The carrier may have a form of a single layer and may also have a form of multilayers. The carrier may be subjected to, as needed, various treatments such as a back-face treatment, an antistatic treatment, and an undercoating treatment, for example. By choosing the right combination of carrier and adhesive, sheets can be made e.g. waterproof, sweatproof or breathable depending on the specific application. Due to the use of this electrically switchable medical adhesive sheet, fixation forces and wear times can be increased while maintaining the same level of skin friendliness and easy peel ability. The controllable trigger also eliminates the risks of accidental debonding and severe damage to the skin.

In general, the voltage source used is not particularly limited as long as it is capable of generating a sufficient potential difference along the thickness direction of the pressure-sensitive adhesive layer to affect a dissociation of the compound into ionic species and migration of the cationic species to the interface between the pressure-sensitive adhesive layer and the adherend.

In embodiments, the pressure-sensitive adhesive sheet or tape according to the present invention may be provided within a kit of parts which further comprise an independent voltage source, which is connected to the conductive members and activated (or deactivated) when debonding (or bonding) is desired.

However, in terms of ease of use, it may be preferred that voltage source is integrated into the pressure-sensitive adhesive sheet or tape.

While not being limited thereto, suitable voltage sources comprise a battery (including primary and secondary batteries, such as lithium-ion batteries; printed batteries, also known as flexible batteries, etc.), an energy harvesting system and/or a wireless power receiver.

In preferred embodiments, one or more battery blocks may be mounted on a flexible PCB and may be coupled using one of a series connection and a parallel connection. In some embodiments, each of the battery blocks may include one or more battery layers, wherein a battery layer may include one of a thin film rechargeable battery, a lithium battery, a sodium-ion battery, and any other suitable type of rechargeable batteries.

Suitable energy harvesting systems are not particularly limited and include devices harvesting electric energy from external ambient sources (e.g. solar power, thermoelectric power, radio frequency).

In especially preferred embodiments, the pressure-sensitive adhesive sheet or tape of the present invention comprises a wireless power receiver. For example, depending on the power requirements, a wireless power receiver based on inductive coupling (such as receivers compatible with the industrial standard Qi or near-field communication (NFC)-antennas) may be used, optionally in combination with a rectifier (e.g. diode- or FET-based), switching regulator, battery charger and battery, for example. In view of the growing number of Qi- and NFC-compatible smartphone devices, patients, physicians and other users may employ their mobile phone in combination with specially designed software to affect electrical debonding of the adhesive sheet or tape in a safe and user-friendly way.

In embodiments, the integrated voltage source and connected components (receivers/antennas, rectifiers, printed circuit boards (PCBs), flexible printed circuits (FPCs), switching regulators, power management unit, etc.) may be independently provided between the adhesive layer and the carrier layer, be positioned next to the adhesive layer, be embedded into the carrier layer, or be arranged between a multiplicity of carrier layers, which may be the same or different.

As has been indicated above, including a debond-on-command trigger allows increasing the adhesive strength beyond levels that are currently employed in the industry. In preferred embodiments, the peel strength of the adhesive tape or sheet of the present invention without applied voltage is preferably more than 1.0 N/10mm, more preferably 1.2 N/10mm or more, further preferably 1.8 N/10mm or more, and particularly preferably 2.0 N/10mm or more, given as an adhesion force in 180° peel test according to EN 1939 (using SUS304 as adherend).

However, when desired, it allows patients/users to deactivate the adhesive by activating the debond trigger, lowering the chance of a painful removal to an absolute minimum. In this respect, the peel strength of the adhesive tape or sheet of the present invention during voltage application is not particularly limited, but is preferably 1.0 N/10mm or less, more preferably 0.1 N/10mm or less, further preferably 0.05 N/10mm or less, and particularly preferably less than 0.01 N/10 mm, given as an adhesion force in 180° peel test according to EN 1939 (using SUS304 as adherend). The adhesion force is an adhesion force in a case where the adhesive sheet is attached to the substrate, is pressed by reciprocating a 2 kg roller two times, and after standing for 30 minutes, is subjected to 180° peeling using a peeling tester during application of a voltage of 10 V.

Moreover, an advantage of this technology is that it promotes re-workability. The debonding effect will be reversible, meaning that once the trigger is removed the adhesion strength will increase again to the previous encountered high levels, giving patients or users a chance to easily reapply the tape. As the force for removal is reduced to an absolute minimum, the chance of removing the top layer of the skin (i.e. stratum corneum) is reduced to almost none which limits the chance of having debris (from the skin) on the exposed adhesive layer. This allows patients to re-use the sheet or tape in a simple and convenient manner.

Except from the additional components described above that facilitate the debonding, the pressure-sensitive adhesive and adhesive sheet or tape according to the present invention will have a similar build-up as conventional sheets, tapes and dressings, and can be applied in the same way, thereby reducing the risks in applying the dressing wrongfully.

Methods of manufacturing the pressure electrically peelable pressure-sensitive adhesive tape or sheet are not particularly limited and may employ techniques commonly used in the preparation of pressure-sensitive adhesive tapes, while the method for forming the conductive member(s) may likewise be appropriately selected from methods known in the art (including, but not limited to printing, plating, vapor deposition, lamination or the like).

### Medical Device and Uses of the Electrically Switchable Pressure-Sensitive Adhesive

Due to the nature of the electrically switchable pressure-sensitive adhesive and adhesive sheet or tape, all medical applications where contact with the skin takes place can make use of the invention. Some examples include wound care, ostomy care, neonatal care and remote patient monitoring. Other uses can relate to any other non-medical application where bonding and debonding of the adhesive sheet to skin might be required, such as toupee products or other cosmetic applications, for example.

In a third embodiment, the present invention generally relates to a medical device comprising electrically peelable pressure-sensitive adhesive for application on skin, comprising: an electrically conductive member, and a pressure-sensitive adhesive layer in direct contact with the electrically conductive member.

Preferably, the medical device comprises an electrically peelable pressure-sensitive adhesive according to the first embodimentas the electrically peelable PSA adhesive, or the pressure-sensitive adhesive sheet or tape according to the second embodiment.

Specific examples of medical devices include, but are not limited to ostomy pouches, glucose test strips, devices for physiological monitoring, sleep diagnostic devices, and therapeutic devices (e.g. sleep apnea treatment devices).

In a preferred embodiment, the medical device is an ostomy appliance (also known as ostomy pouch). In ostomy care, the adhesive plays an important role in pouch performance by supporting the ostomy bag and protecting the peristomal skin from being exposed to stomal effluent. The composition of the adhesive is very important in the overall performance of the appliance, allowing it to adhere safely to the skin and absorb moisture during wear. When the ostomy adhesive is permanently attached to the bag, the ostomy appliance is conventionally limited to be worn for short periods of time (typically up to three days) since the adhesive must be optimized for ease of removal. On the other hand, so-called two-piece products, wherein the adhesive is attached to the bag via a coupling system that can be separated, the ostomy appliance is designed for longer wear time

(typically up to six days), so that the adhesive is optimized for high adhesion and erosion resistance. However, the removal of such a two-piece ostomy appliance is often a very painful process as it may involve stripping of the skin as well as skin irritation and trauma. In addition, many adhesives tend to leave residues on the skin upon removal. By using the electrically peelable pressure-sensitive adhesive according to the first embodiment or the pressure-sensitive adhesive sheet or tape according to the second embodiment in an ostomy appliance, the usability of both one- and two-piece products may be significantly prolonged and the confidence into the adhesive may be significantly improved while entirely eliminating the pain during removal.

A fourth embodiment of the present invention relates to the use of the electrically peelable pressure-sensitive adhesive according to the first embodiment in medical pressure-sensitive adhesive sheets or tapes, wound dressings, band aids, transdermal patches or for affixing medical devices (e.g. ostomy pouches, glucose test strips, devices for physiological monitoring (e.g. EKG electrodes), sleep diagnostic devices, respiratory devices and therapeutic devices), wearables (e.g. sensor attachment for tracking pulse or health indicators, mounting of discrete microphones) and cosmetic products (e.g. toupees, special effects make-up) onto skin.

### Method of Applying the Pressure-Sensitive Adhesive Sheet or Tape on Skin

In a fifth aspect, the present invention relates to a method of applying a pressure-sensitive adhesive sheet or tape according to the second embodiment or a medical device according to the third embodiment on skin, comprising the subsequent steps of: (a) adhering the pressure-sensitive adhesive sheet or tape according to the second embodiment or the medical device according to the third embodiment on skin via the pressure-sensitive adhesive layer and establishing contact between the one or more additional conductive members and the skin surface, wherein a voltage source is either integrated into the medical device or pressure-sensitive adhesive sheet or tape or independently provided and connected so that a first electrode of the voltage source is connected with the electrically conductive member and a second electrode of the voltage source is connected with the one or more additional conductive members, the first and second electrodes having opposite polarities; (b) activating the voltage source so as to establish a potential difference of 1 V to 100 V along the thickness direction of the pressure-sensitive adhesive layer; (c) peeling off the medical device or pressure-sensitive adhesive sheet or tape from the skin surface; and (d) optionally re-adhering the medical device or pressure-sensitive adhesive sheet or tape to the skin and subsequently deactivating or adjusting the voltage source to establish a potential difference of less than 1 V along the thickness direction of the pressure-sensitive adhesive layer.

It is understood that in step a), electrical contact between the one or more additional conductive members and the skin surface may be established directly or indirectly (e.g., via a conductive gel or liquid).

As has been set out above in combination with the first embodiment, the voltage to be applied is not particularly limited as long as the adhesive sheet can be peeled off. However, a DC voltage in the range of from 1 to 20 V, more preferably from 1 to 10 V, especially preferably 1 to 5 V is preferred.

The duration of step b) is not particularly limited, as long as peeling is possible, but is preferably between 1 second or longer and 2 minutes, further preferably between 1 second and 60 seconds. An upper limit of the application time is more preferably 40 seconds and further preferably 20 seconds.

In step c), the peeling is preferably performed while maintaining the potential difference in the range of 1 V to 100 V (further preferably from 1 to 20 V, more preferably from 1 to 10 V, especially preferably 1 to 5 V), which may be either during a time when the voltage source is still activated or shortly after stopping the voltage. In the latter case, peeling is preferably performed within 60 seconds, and more preferably within 30 seconds after deactivating the voltage source, since otherwise the adhesion force of the adhesive sheet may recover and the adhesive strength may be increased again.

It will be understood that the preferred features of the first to fifth embodiments may be freely combined in any combination, except for combinations where at least some of the features are mutually exclusive.

For instance, the present invention may relate to the following aspects:
1. An electrically peelable pressure-sensitive adhesive for application on skin, comprising:
   an electrically conductive member, and
   a pressure-sensitive adhesive layer in direct contact with the electrically conductive member,
   wherein the pressure-sensitive adhesive layer comprises a compound which dissociates into ionic species upon applying a voltage in the range of from 1 to 100 V to the electrically conductive member and wherein the pressure-sensitive adhesive layer exhibits an ionic conductivity of at least 10⁻¹¹ S/cm at 25°C with respect to the dissociated ionic species.
2. The electrically peelable pressure-sensitive adhesive according to aspect 1, wherein the compound is an ionic liquid.
3. The electrically peelable pressure-sensitive adhesive according to any of aspects 1 or 2, wherein the ionic liquid comprises a cation selected from a nitrogen-containing onium cation, a sulfur-containing onium cation, or a phosphorus-containing onium cation, and preferably from an imidazolium-based cation, an ammonium-based cation or a pyridinium-based cation, and/or wherein the compound dissociates into ionic species upon applying a voltage in the range of from 1 to 10 V to the electrically conductive member.
4. The electrically peelable pressure-sensitive adhesive according to any of aspects 1 to 3, wherein the pressure-sensitive adhesive layer exhibits an ionic conductivity of at least 10⁻⁷ S/cm at 25°C and preferably at least 10⁻⁶ S/cm with respect to the dissociated ionic species.
5. The electrically peelable pressure-sensitive adhesive according to any of aspects 1 to 4, wherein the ionic species comprise a cation having a molecular weight of 250 g/mol or less, preferably 200 g/mol or less, and particularly preferably 160 g/mol or less.
6. The electrically peelable pressure-sensitive adhesive according to any of aspects 1 to 5, wherein the pressure-sensitive adhesive layer comprises an adhesive (co-)polymer selected from one or more of an acrylic (co-)polymer, a silicone-based (co-)polymer or a urethane-based polymer, and wherein a content of the compound which dissociates into ionic species is 0.5 to 30 parts by weight with respect to 100 parts by weight of the adhesive (co-)polymer.
7. The electrically peelable pressure-sensitive adhesive according to any of aspects 1 to 6, wherein the electrically conductive member comprises a metal, metal oxide, carbon and/or a conductive polymer.
8. Pressure-sensitive adhesive sheet or tape for application on skin, comprising:
   the electrically peelable pressure-sensitive adhesive according to any of aspects 1 to 7, and
   one or more additional conductive members that are not in electrical contact with any of the pressure-sensitive adhesive layer and the electrically conductive member, wherein the one or more additional conductive members are provided so as to establish electrical contact to the skin upon applying the sheet or tape.
9. The pressure-sensitive adhesive sheet or tape according to aspect 8, further comprising a voltage source, wherein a first electrode thereof is connected to the electrically conductive member and a second electrode thereof is connected to the one or more additional conductive members, the first and second electrodes having opposite polarities.
10. The pressure-sensitive adhesive sheet or tape according to aspect 9, wherein the voltage source comprises a battery, an energy harvesting system and/or a wireless power receiver.
11. The pressure-sensitive adhesive sheet or tape according to any of aspects 8 to 10, further comprising a carrier layer comprising any of polyethers, polyester polyurethanes, thermoplastic polyester elastomers, polyethylene, polypropylene, polyethylene terephthalate, medical grade woven materials or medical grade non-woven materials.
12. Medical device comprising the electrically peelable pressure-sensitive adhesive according to any of aspects 1 to 7 or the pressure-sensitive adhesive sheet or tape according to aspects 8 to 11.
13. Use of the electrically peelable pressure-sensitive adhesive according to aspects 1 to 7 in medical pressure-sensitive adhesive sheets or tapes, transdermal patches, wound dressings, band aids, or for affixing medical devices, wearables and cosmetic products onto skin.
14. Method of applying a pressure-sensitive adhesive sheet or tape on skin, comprising the subsequent steps of:
   (a) adhering the pressure-sensitive adhesive sheet or tape according to any of aspects 8 to 11 on skin via the pressure-sensitive adhesive layer and establishing contact between the one or more additional conductive members and the skin surface, wherein a voltage source is either integrated into the pressure-sensitive adhesive sheet or tape or independently provided and connected so that a first electrode of the voltage source is connected with the electrically conductive member and a second electrode of the voltage source is connected with the one or more additional conductive members, the first and second electrodes having opposite polarities;
   (b) activating the voltage source so as to establish a potential difference of 1 V to 100 V along the thickness direction of the pressure-sensitive adhesive layer;
   (c) peeling off the pressure-sensitive adhesive sheet or tape from the skin surface;
      and
   (d) optionally re-adhering the pressure-sensitive adhesive sheet or tape to the skin and subsequently deactivating or adjusting the voltage source to establish a potential difference of less than 1 V along the thickness direction of the pressure-sensitive adhesive layer.

Once given the above disclosure, many other features, modifications, and improvements will become apparent to the skilled artisan.

### REFERENCE NUMERALS

- A:: electrically peelable pressure-sensitive adhesive
1: pressure-sensitive adhesive layer
2: first conductive member
3: skin
4a: second conductive member
4b: third conductive member
5: spacer
6: voltage source

## Claims

1. A medical device, comprising an electrically peelable pressure-sensitive adhesive for application on skin, comprising:
an electrically conductive member, and
a pressure-sensitive adhesive layer in direct contact with the electrically conductive member.

2. A pressure-sensitive adhesive sheet or tape for application on skin, comprising:
an electrically peelable pressure-sensitive adhesive comprising:
an electrically conductive member, and
a pressure-sensitive adhesive layer in direct contact with the electrically conductive member, and
one or more additional conductive members that are not in electrical contact with any of the pressure-sensitive adhesive layer and the electrically conductive member, wherein the one or more additional conductive members are provided so as to establish electrical contact to the skin upon applying the sheet or tape.

3. The medical device according to claim 1 or the pressure-sensitive adhesive sheet or tape for application on skin according to claim 2,wherein the pressure-sensitive adhesive layer comprises a compound which dissociates into ionic species upon applying a voltage in the range of from 1 to 100 V to the electrically conductive member and wherein the pressure-sensitive adhesive layer exhibits an ionic conductivity of at least 10⁻¹¹ S/cm at 25°C with respect to the dissociated ionic species.

4. The medical device or pressure-sensitive adhesive sheet or tape for application on skin according to claim 3, wherein the compound is an ionic liquid.

5. The medical device or pressure-sensitive adhesive sheet or tape for application on skin according to claim 4, wherein the ionic liquid comprises a cation selected from a nitrogen-containing onium cation, a sulfur-containing onium cation, or a phosphorus-containing onium cation, and preferably from an imidazolium-based cation, an ammonium-based cation or a pyridinium-based cation, and/or wherein the compound dissociates into ionic species upon applying a voltage in the range of from 1 to 10 V to the electrically conductive member.

6. The medical device or pressure-sensitive adhesive sheet or tape for application on skin according to any of claims 3 to 5, wherein the pressure-sensitive adhesive layer exhibits an ionic conductivity of at least 10⁻⁷ S/cm at 25°C and preferably at least 10⁻⁶ S/cm with respect to the dissociated ionic species.

7. The medical device or pressure-sensitive adhesive sheet or tape for application on skin according to any of claims 3 to 6, wherein the ionic species comprise a cation having a molecular weight of 250 g/mol or less, preferably 200 g/mol or less, and particularly preferably 160 g/mol or less.

8. The medical device or pressure-sensitive adhesive sheet or tape for application on skin according to any of claims 3 to 7, wherein the pressure-sensitive adhesive layer comprises an adhesive (co-)polymer selected from one or more of an acrylic (co-)polymer, a silicone-based (co-)polymer or a urethane-based polymer, and wherein a content of the compound which dissociates into ionic species is 0.5 to 30 parts by weight with respect to 100 parts by weight of the adhesive (co-)polymer.

9. The medical device according to any of claims 1 to 8 or the pressure-sensitive adhesive sheet or tape according to any of claims 2 to 8, wherein the electrically conductive member comprises a metal, metal oxide, carbon and/or a conductive polymer.

10. The medical device according to any of claims 1 to 9 or the pressure-sensitive adhesive sheet or tape according to any of claims 2 to 9, further comprising a voltage source, wherein a first electrode thereof is connected to the electrically conductive member and a second electrode thereof is connected to the one or more additional conductive members, the first and second electrodes having opposite polarities.

11. The medical device or pressure-sensitive adhesive sheet or tape according to claim 10, wherein the voltage source comprises a battery, an energy harvesting system and/or a wireless power receiver.

12. The medical device according to any of claims 1 to 11 or the pressure-sensitive adhesive sheet or tape according to any of claims 2 to 11, further comprising a carrier layer comprising any of polyethers, polyester polyurethanes, thermoplastic polyester elastomers, polyethylene, polypropylene, polyethylene terephthalate, medical grade woven materials or medical grade non-woven materials.

13. An electrically peelable pressure-sensitive adhesive for application on skin, comprising:
an electrically conductive member, and
a pressure-sensitive adhesive layer in direct contact with the electrically conductive member,
wherein the pressure-sensitive adhesive layer comprises a compound which dissociates into ionic species upon applying a voltage in the range of from 1 to 100 V to the electrically conductive member and wherein the pressure-sensitive adhesive layer exhibits an ionic conductivity of at least 10⁻¹¹ S/cm at 25°C with respect to the dissociated ionic species.

14. Use of the electrically peelable pressure-sensitive adhesive according to claim 13 in medical pressure-sensitive adhesive sheets or tapes, transdermal patches, wound dressings, band aids, or for affixing medical devices, wearables and cosmetic products onto skin.

15. Method of applying a medical device or pressure-sensitive adhesive sheet or tape on skin, comprising the subsequent steps of:
(a) adhering the medical device according to any one of claims 1 to 12 or a pressure-sensitive adhesive sheet or tape according to any of claims 2 to 12 on skin via the pressure-sensitive adhesive layer and establishing contact between the one or more additional conductive members and the skin surface, wherein a voltage source is either integrated into the medical device or pressure-sensitive adhesive sheet or tape or independently provided and connected so that a first electrode of the voltage source is connected with the electrically conductive member and a second electrode of the voltage source is connected with the one or more additional conductive members, the first and second electrodes having opposite polarities;
(b) activating the voltage source so as to establish a potential difference of 1 V to 100 V along the thickness direction of the pressure-sensitive adhesive layer;
(c) peeling off the medical device or pressure-sensitive adhesive sheet or tape from the skin surface; and
(d) optionally re-adhering the medical device or pressure-sensitive adhesive sheet or tape to the skin and subsequently deactivating or adjusting the voltage source to establish a potential difference of less than 1 V along the thickness direction of the pressure-sensitive adhesive layer.
